# EUROPÄISCHE PATENTANMELDUNG

(11) **EP 2 246 078 A1**
(43) Veröffentlichungstag der Anmeldung: **03.11.2010**
(21) Anmeldenummer: 09075211.4
(22) Anmeldetag: 29.04.2009
(51) Int. Cl.: A61M 1/10, F16C 1/06, F16C 1/24

(54) **Wellenanordnung mit einer Welle, die innerhalb einer fluidgefüllten Hülle verläuft**

(71) Anmelder: ECP Entwicklungsgesellschaft mbH, 12247 Berlin (DE)
(72) Erfinder: Liebing, Reiner, 12587 Berlin (DE)
(74) Vertreter: Pfenning, Meinig & Partner GbR

(57) **Zusammenfassung**

Bei einer Wellenanordnung mit einer Welle (7), die innerhalb einer fluidgefüllten Hülle (1) verläuft und die mittels eines Antriebs (8) von außerhalb der Hülle antreibbar ist, wobei die Welle (7) an ihrer Mantelfläche eine Oberflächenstruktur aufweist, die bei Rotation das Fluid in einer Stromrichtung (30) in Längsrichtung der Welle fördert, ist gemäß der Erfindung eine mit der Welle drehbare Hülse (19) vorgesehen, die wenigstens ein Förderelement (20) zur Förderung des Fluids in einer der Stromrichtung entgegengesetzten Gegenstromrichtung aufweist. Hierdurch wird eine blasenfreie Förderung des Fluids entlang der Welle (7) erleichtert.

## Beschreibung

Die Erfindung liegt auf dem Gebiet der Mechanik bzw. des Maschinenbaus und ist mit besonderem Vorteil in der Feinwerktechnik, das heißt beim Bau kleiner Maschinen und Geräte, einsetzbar.

Besondere Vorteile bringt der Einsatz in der Medizintechnik, wo häufig, insbesondere bei der minimalinvasiven Medizin, Bewegungen über Wellen, insbesondere biegsame Wellen, durch kleine Öffnungen bzw. körpereigene Gefäße unter besonders schwierigen Umgebungsbedingungen übertragen werden müssen.

Eine besondere Anwendungsform stellt der Antrieb von Flüssigkeitspumpen in Mikrobauform dar, die beispielsweise als Herzpumpen Anwendung finden und die mit einem Herzkatheter an ihren Betriebsort, beispielsweise eine Herzkammer, gebracht werden können. Dazu müssen nicht nur die entsprechenden Pumpen sehr geringe Baugröße aufweisen, sondern auch für die Übertragung der Pumpleistung über eine Welle liegen schwierige Bedingungen vor. Die Welle verläuft üblicherweise durch einen Herzkatheter und wird vom Körperäußeren her durch eine Durchführung angetrieben. Am distalen Ende des Katheters wird die Bewegung auf die Pumpe übertragen. Derartige Wellen sind üblicherweise biegsam, so dass besonders bei den hohen benötigten Drehzahlen intensive Verformungen stattfinden. Daher stellt es nicht nur besonders hohe Anforderungen, die hohen Drehzahlen zu erreichen, sondern ebenso, die entsprechende Wärme, die durch die Verformung der Welle entsteht, abzuführen.

Ein entsprechender Herzkatheter ist üblicherweise mit einer körperverträglichen Flüssigkeit gefüllt, um einerseits die Welle zu schmieren und andererseits zu kühlen.

Da entsprechende Wellen üblicherweise, um ihre Biegsamkeit zu fördern, aus dünneren Strängen zusammengesetzt und verdrillt sind, ergibt sich eine spindelförmige Oberflächenstruktur, die bei schneller Drehung zur Förderung des in dem Katheter befindlichen Fluids längs der Welle führt. Dieser Effekt ist im Allgemeinen unerwünscht, da er ein Druckgefälle des Fluids in dem Katheter schafft. Von dem antriebsseitigen Ende des Katheters her muss neues Fluid nachströmen. Ist dieses nicht verfügbar, so können entweder durch die Wellendurchführung oder auch durch eine Entlüftungsöffnung, die ebenfalls im antriebsnahen Bereich des Katheters vorgesehen sein kann und die zum Entlüften des Katheters dient, unerwünschte Flüssigkeiten oder Gase, beispielsweise Luft, angesaugt werden.

Üblicherweise ist eine Zuführung für das in dem Katheter befindliche Fluid vorgesehen, durch die das Fluid mittels einer Pumpvorrichtung nachgepumpt wird. Es hat dabei Vorteile, eine nicht zu große Menge des Fluids zu pumpen, d. h. einen sehr geringen Volumenstrom zu erzeugen. Liefert jedoch die Pumpe der Fluidzuführung keinen ausreichenden Volumenstrom, so entsteht im Bereich des Fluidzuflusses durch die Absaugtendenz der Welle ein Unterdruck, der unerwünscht ist.

Der vorliegenden Erfindung liegt daher die Aufgabe zugrunde, bei einer Wellenanordnung der eingangs genannten Art eine bestimmungsgemäße blasenfreie Füllung der Wellenanordnung mit dem vorgesehenen Fluid zu ermöglichen.

Die Aufgabe wird gemäß der Erfindung mit den Merkmalen des Patentanspruchs 1 gelöst.

Es ist eine die Welle umgebende, beispielsweise im antriebsseitigen Endbereich der Welle angeordnete, mit dieser rotierende Hülse, eine sogenannte Gegenstromhülse, vorgesehen, die wenigstens ein Förderelement zur Förderung des Fluids in einer der Stromrichtung entgegengesetzten Gegenstromrichtung aufweist.

Beispielsweise kann die Hülse fest mit der Welle verbunden sein und entsprechend mit gleicher Drehzahl wie diese rotieren. Die Hülse kann auf ihrer Außenseite eine wendelartige Schaufelblattstruktur oder einzelne Schaufelblätter oder Stege in entsprechender schräg gestellter oder wendelförmiger Aufbaustruktur aufweisen. Wichtig dabei ist, dass die Hülse eine Strömung bzw. einen Druckanstieg entgegen der Stromrichtung, d.h. in Richtung des Wellenendes bzw. zum Anschlussraum hin erzeugt. Dadurch wird ein Unterdruck im Anschlussraum und das Einströmen von Luft durch eine Entlüftungsöffnung oder die Wellendurchführung wirksam verhindert. Auch ein übermäßiges Nachsaugen des Fluids, falls dies durch einen Fluidzufluss nachgeliefert wird, wird vermieden. Durch die Hülse wird zum antriebsseitigen Wellenende hin der Druck erhöht, während er auf der antriebsfernen Seite der Hülse durch das Ansaugen etwas abgesenkt wird. In diesem Bereich wirken die Förderrichtungen der Welle einerseits und der Hülse andererseits gegeneinander, so dass dort tendenziell der Druck abgesenkt wird. In diesem Bereich befindet sich jedoch kein Zufluss und keine Öffnung, so dass dort die Entstehung von Gasblasen vermieden werden kann.

Damit bewirkt die Erfindung, dass die Welle zuverlässig mit hohen Drehzahlen betrieben werden kann, ohne dass die Fluidkühlung und/oder -schmierung aussetzt und ohne dass Gasblasen entlang der Welle zum antriebsfernen Ende eines Katheters gefördert werden.

Auch bei anderen Einsätzen im nichtmedizinischen Bereich ist eine derartige Blasenbildung in einem die Welle umgebenden Fluid unerwünscht und kann mit den Mitteln der Erfindung verhindert werden.

Eine vorteilhafte Ausgestaltung der Erfindung sieht vor, dass die Hülse in Stromrichtung gesehen hinter einem Anschlussraum angeordnet ist, in den eine Durchführung der Welle oder eines Wellenantriebs mündet.

In dem Anschlussraum, der üblicherweise im antriebsseitigen Endbereich der Welle angeordnet ist, kann auch zusätzlich oder alternativ zu einer Wellendurchführung eine Fluidzuführung für das die Hülle füllende Fluid und/oder eine Entlüftungsöffnung zur Entlüftung des eingefüllten Fluids angeordnet sein.

Vorteilhaft ist in jedem Fall, dass mittels der Hülse im Betrieb ein Druckabfall im Anschlussraum verhindert wird, um dort das Eindringen von Gasblasen oder anderen unerwünschten Fluiden oder das übermäßige Nachsaugen des Fluids, mit dem die Hülle gefüllt ist, zu verhindern. Durch die Anordnung der Hülse hinter dem Anschlussraum in Stromrichtung gesehen wirken dort, d. h. in einem Bereich, der vom antriebsseitigen Ende und dem Anschlußraum der Welle üblicherweise ein Stück beabstandet ist, die Pumpwirkungen der Hülse und der Welle gegeneinander, so dass in diesem Bereich, außerhalb des Anschlussraums, der Druck des Fluids abgesenkt wird, wo üblicherweise keine Öffnungen in der Hülle vorgesehen sind und somit keine unerwünschten Stoffe angesaugt werden können.

Um eine angemessene Führung der Hülse bei der Rotationsbewegung zu gewährleisten und je nach der Art der Kopplung zwischen der Hülse und der Welle auch unerwünschte Interaktionen zwischen diesen zu verhindern, kann vorteilhaft eine Lagerung der Hülse an ihrem äußeren Umfang, insbesondere mittels eines Wälzlagers, vorgesehen sein. Es kann dort jedoch wahlweise auch ein Magnetlager oder ein gut geschmiertes Gleitlager vorgesehen sein.

Je nach Art des Lagers kann auch ein weiteres Lager zur Stützung der Hülse vorteilhaft vorgesehen sein.

Die Welle weist vorteilhaft zur Erzielung der Pumpwirkung in ihrer Längsrichtung an ihrer Oberfläche eine spindelförmige Außenkontur auf. Diese bewirkt bei einer Rotation, insbesondere bei Drehzahlen zwischen 20.000 und 40.000 Umdrehungen pro Minute, eine Förderung des Fluids in Längsrichtung zusätzlich zu etwa noch auftretenden radialen und azimutalen Bewegungsrichtungen des Fluids. Diese Pumpwirkung der Welle reicht aus, um eine kontinuierliche Kühlung und Schmierung zu gewährleisten. Die Welle kann beispielsweise aus einem Bündel von verdrillten Strängen, z. B. Drähten oder Fasern, insbesondere Glasfasern, bestehen und bereits hierdurch die spindelförmige Außenkontur aufweisen. Die Verdrillung der einzelnen Stränge ist üblicherweise derart gerichtet, dass sie mit der natürlichen Verdrillung durch Aufbringen eines Drehmoments am antriebsseitigen Ende übereinstimmt, so dass im Betrieb die Verdrillung der Welle stabilisiert wird. Dieser Verdrillungssinn stimmt mit der für die Pumprichtung zum antriebsfernen Ende notwendigen spindelförmigen Kontur überein.

Vorteilhaft kann die Hülle der Welle an ihrem dem Antrieb fernen Ende einen Auslass für das Fluid aufweisen. In diesem Fall kann dort das Fluid entweder ausgelassen werden, beispielsweise auch durch eine Durchführung der Welle, z. B. wenn diese in ein nachfolgend angeordnetes Pumpengehäuse weiterführt, oder das Fluid kann an dem Auslass abgepumpt und zurückgeführt werden.

In jedem Fall wird das Fluid im Falle einer Verwendung im medizinischen Bereich ein körperverträgliches Fluid wie beispielsweise eine Kochsalzlösung sein, so dass eventuell ins Körpergewebe oder in ein Blutgefäß eintretende Flüssigkeitsmengen keinen schädlichen Einfluss haben.

An dem entsprechenden Auslass kann das Fluid auch aktiv abgesaugt werden, um einen unbeabsichtigten Austritt durch Öffnungen, wie beispielsweise eine Wellendurchführung, zu verhindern.

Die Erfindung bezieht sich außer auf eine Wellenanordnung der beschriebenen Art auch auf ein Verfahren zum Betrieb einer derartigen Wellenanordnung, bei dem die Welle mit einer Geschwindigkeit von mindestens 300 Umdrehungen pro Minute rotiert. Das vorteilhafte Verfahren kann außerdem vorsehen, dass die Welle mit einer Geschwindigkeit von nicht mehr als 40.000 Umdrehungen pro Minute rotiert.

Zudem kann vorgesehen sein, dass zur Kühlung und Schmierung der Welle ein konstanter Fluss des die Hülle füllenden Fluids in den Anschlussraum gepumpt wird. Dabei kann je nach Einrichtung der Speisepumpe ein konstanter Volumenstrom eingepumpt werden, oder es kann in dem Anschlussraum ein konstanter Druck des Fluids aufrechterhalten werden. Üblicherweise werden sich beim Betrieb der Welle nach kurzer Zeit stabile Betriebsparameter einstellen.

Das erfindungsgemäße Verfahren kann außerdem vorsehen, dass das Fluid an einem Auslass an dem antriebsfernen Ende aus der Hülle der Welle abgesaugt wird.

Im Folgenden wird die Erfindung anhand eines Ausführungsbeispiels in einer Zeichnung gezeigt und anschließend beschrieben.

Dabei zeigt
- Fig. 1: eine schematische Übersicht über einen Ka- theter mit einer Welle, die zu einer in ei- ne Herzkammer eingebrachten Pumpe führt,
- Fig. 2: schematisch einen Längsschnitt durch den antriebsseitigen Endbereich eines Katheters mit einer eingezogenen Antriebswelle und
- Fig. 3: eine Ansicht eines distalen Endes eines Ka- theters mit einer Welle.

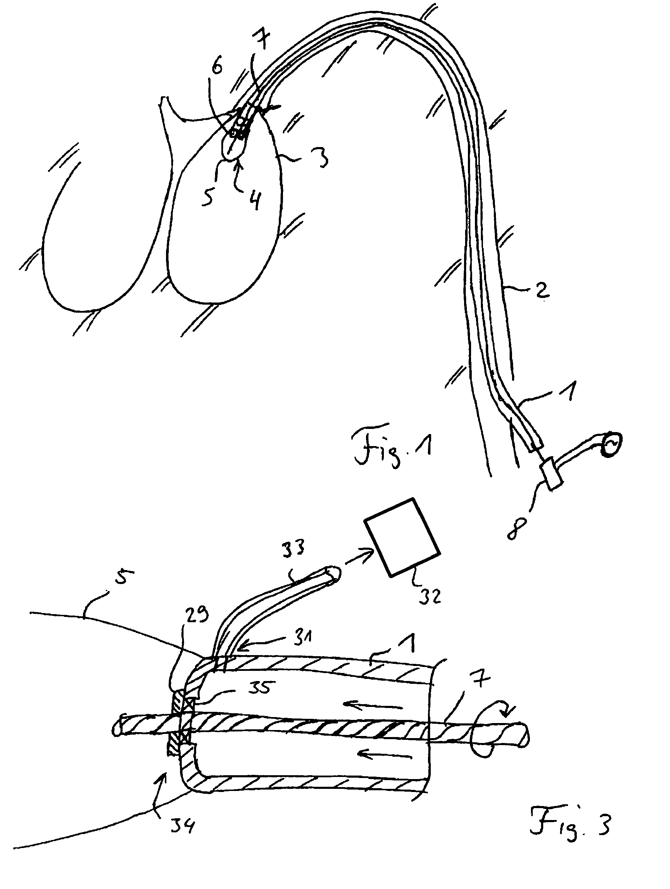

Fig. 1 zeigt eine typische Anwendung der erfindungsgemäßen Wellenanordnung im medizintechnischen Bereich im Zusammenhang mit einer Herzpumpe. Hierbei ist ein Katheter 1 vorgesehen, der durch ein Blutgefäß bis in eine Herzkammer 3 geschoben wird und der an seinem distalen Ende eine Mikropumpe 4 aufweist. Diese verfügt über ein Gehäuse 5, in dem ein Rotor 6 mittels einer Welle 7 mechanisch rotierend antreibbar ist.

Die Welle 7 ist durch eine Durchführung des Pumpengehäuses 5 in den Katheter 1 dichtend durchgeführt und durch den Katheter 1 bis zu einem außerhalb des Körpers liegenden Wellenantrieb in Form eines Motors 8 geführt.

Üblicherweise sind das Pumpengehäuse 5 und der Rotor 6 bei einer solchen Herzkatheterpumpe derart ausgebildet, dass sie zum Einführen durch das Blutgefäß in die Herzkammer komprimierbar und innerhalb der Herzkammer expandierbar sind. Zum Entfernen der Pumpe aus dem Körper wird diese wiederum komprimiert, um durch das Gefäß mittels des Katheters zurückgezogen werden zu können.

Der dargestellte Katheter ist sehr biegsam, um ohne Verletzung des Blutgefäßes durch dessen Biegungen, insbesondere den Aortenbogen, hindurchführbar zu sein. Entsprechend muss auch die Welle 7, die innerhalb des als Hülle 1 wirkenden Katheters verläuft, biegsam sein. Dies wird üblicherweise dadurch erreicht, dass die Welle entweder aus einem sehr biegsamen Material besteht und einstückig aufgebaut ist, beispielsweise als Glas- oder Kunststofffaser, oder dass sie aus verschiedenen Strängen geringerer Dicke durch Verseilung oder Verdrillung aufgebaut ist.

Im Falle einer einstückigen Herstellung kann bei der Herstellung eine entsprechende Oberflächenstruktur vorgesehen werden, die zumindest bei hohen Drehzahlen der Welle eine Förderung eines Fluids in Längsrichtung bewirkt. Bei der Herstellung durch Verdrillung ergibt sich eine derartige Struktur ohnehin und kann für die Fluidförderung genutzt werden.

In jedem Fall ergibt sich bei hohen Drehzahlen der Welle, also insbesondere zwischen 20.000 und 40.000 Umdrehungen pro Minute, eine hohe Verformungsgeschwindigkeit der Welle und gegebenenfalls auch Reibung zwischen den einzelnen Elementen, die zur Erwärmung der Welle führt. Aus diesem Grunde ist die Einbettung der Welle in eine Flüssigkeit sinnvoll, die einerseits Wärme abführt und andererseits für einen Schmierfilm zwischen verschiedenen Elementen der Welle 7 oder auch zwischen der Welle und einer diese umgebenden Führung, beispielsweise einer flexiblen Drahtwendel, sorgt.

Eine derartige Führung kann beispielsweise in einer Wendel 9, bestehen, deren Innendurchmesser größer als der der Welle ist und die in dem Katheter befestigt oder lose um die Welle herumgelegt ist. Sie ist ebenso biegbar wie der Katheter und stellt eine definierte Kontakt- und Reibungsfläche für die Welle her.

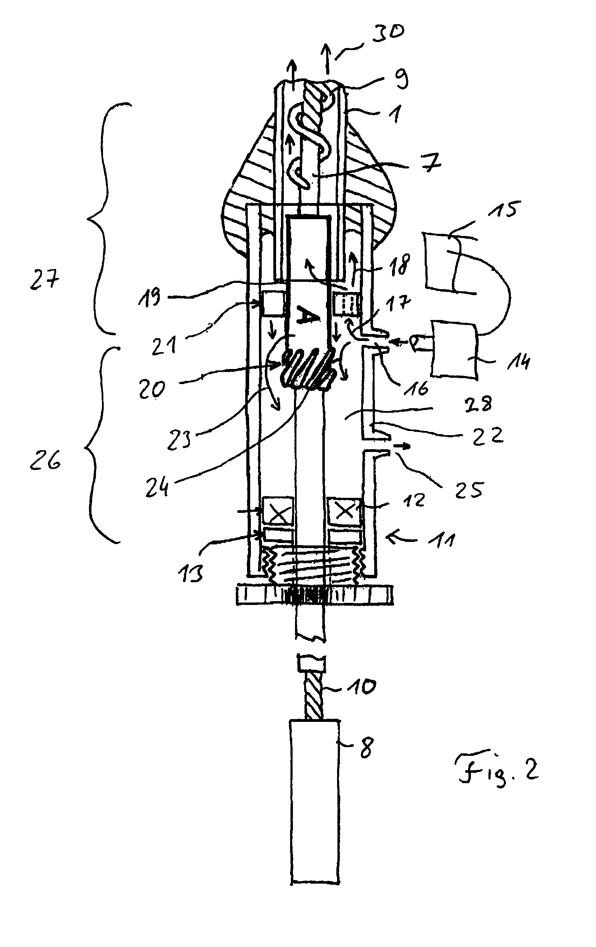

Fig. 2 zeigt in stark vergrößertem Maßstab das antriebsseitige Ende der Welle 7, die in der Wendel 9 innerhalb der Hülle/des Katheters 1 verläuft.

Es ist ein Motor 8 dargestellt, der eine Motorwelle 10 aufweist, die unmittelbar mit der Welle 7 identisch oder an diese angekoppelt sein kann. Zu diesem Zweck kann eine der Wellen 7, 10 durch eine Durchführung 11 mit einem Lager 12 und einer Stopfbuchse 13 in den Anschlussraum 28 durchgeführt sein. Eine derartige Durchführung kann weitestgehend dicht, jedoch nicht vollkommen dicht ausgeführt sein.

Des Weiteren zeigt die Fig. 2 eine Förderpumpe 14, die aus einem Reservoir 15 das Fluid fördert, das innerhalb des Katheters 1 die Welle 7 umgibt und dieses durch einen Zufluss 16 in den Anschlussraum 28 fördert. Von hier aus wird das Fluid, wie mittels der Pfeile 17, 18 dargestellt, entlang der Welle 7 in Stromrichtung 30 zum distalen Ende des Katheters 1 hin befördert, sofern die Welle 7 sich in schneller Rotation befindet und entsprechend mit der spiralförmigen Außenkontur das Fluid fördert.

Es ist zudem eine Hülse 19 dargestellt, die Förderelemente 20 an ihrem Umfang aufweist, die im dargestellten Fall als spiralig angeordnete Förderschaufeln ausgeprägt sind.

Die Hülse 19 ist in einem Lager 21 radial außen gegenüber dem Anschlussraumgehäuse 22 drehbar gelagert. Sie ist nach innen mit der Welle 7 verbunden, beispielsweise über Streben oder über einen Verguss.

Durch Rotation der Hülse 19 und die Funktion der Förderelemente 20 wird eine Strömung des Fluids in Richtung der Pfeile 23, 24 erreicht, und zwar in den Anschlussraum 28 hinein. Dadurch wird im Anschlussraum 28 der Druck des Fluids leicht erhöht. Es wird hierdurch sichergestellt, dass das Ansaugen von Fremdflüssigkeiten oder Gas bzw. Luft sowohl durch die Durchführung 11 als auch durch eine Belüftungsöffnung 25 oder den Zufluss 16 verhindert wird.

Durch die kombinierte Wirkung der Hülse 19 und der fördernden Welle 7 wird in dem mit der geschweiften Klammer 26 bezeichneten Bereich der Fluiddruck leicht erhöht, während er durch beidseitiges Absaugen in dem Bereich der geschweiften Klammer 27 leicht gesenkt wird. Dort kann jedoch kein unerwünschtes Fluid oder Gas angesaugt werden, so dass insgesamt die Bildung von Blasen oder das Eindringen von Fremdfluiden in den Katheter 1 durch die Merkmale der Erfindung verhindert wird. Die Pumpleistung der Hülse 19 ist durch die Gestaltung der Förderelemente 20 derart zu steuern, dass eine Saldopumpleistung der Welle im Betrieb bestehen bleibt, die einen mäßigen Fluss des Fluids entlang der Welle 7 zu Kühlungs- und Schmierungszwecken gewährleistet.

Fig. 3 zeigt das distale oder dem Antrieb abgewandte Ende der Welle 7 mit einer Durchführung 34 aus dem Katheter in das Pumpengehäuse 5. Die Durchführung 34 sieht eine Stopfbuchse 29 und ein Lager 35 vor, das als Gleitlager ausgebildet sein kann.

Um einen Fluidfluss zu gewährleisten und das Austreten des Fluids durch die Durchführung 34 zu verhindern oder zu verringern, wird das Fluid durch eine Abflussöffnung 31 mittels einer Absaugpumpe 32 über eine Abflussleitung 33 abgesaugt. Es kann dann wieder dem Reservoir 15 zugeführt oder gänzlich entfernt werden.

Obwohl die Erfindung in einer speziellen Anwendung im medizintechnischen Bereich beschrieben wurde, ist sie universell einsetzbar, insbesondere bei verschiedensten Anwendungen biegsamer Wellen, z. B. bei Werkzeugen zur spanenden Bearbeitung von Bauteilen, wo eine Kühlung und Schmierung der Welle durch ein Fluid wichtig sein kann.

## Patentansprüche

1. Wellenanordnung mit einer Welle (7), die innerhalb einer mit einem Fluid gefüllten Hülle (1) verläuft und die mittels eines Antriebs (8) insbesondere von außerhalb der Hülle (1) antreibbar ist, wobei die Welle (7) an ihrer Mantelfläche eine Oberflächenstruktur aufweist, die bei Rotation das Fluid in einer Stromrichtung in Längsrichtung der Welle fördert, **gekennzeichnet durch** eine im Bereich der Welle angeordnete, die Welle umgebende und mit dieser rotierende Hülse (19), die wenigstens ein Förderelement (20) zur Förderung des Fluids in einer der Stromrichtung (30) entgegengesetzten Gegenstromrichtung (24) aufweist.

2. Wellenanordnung nach Anspruch 1, **dadurch gekennzeichnet, dass** die Hülse (19) in Stromrichtung (30) gesehen hinter einem Anschlussraum (28) angeordnet ist, in den eine Durchführung (11) der Welle (7) oder eines Wellenantriebs mündet.

3. Wellenanordnung nach Anspruch 1 oder 2, **dadurch gekennzeichnet, dass** die Hülse (19) in Stromrichtung (30) gesehen hinter einem Anschlussraum (28) angeordnet ist, in den eine Fluidzuführung (16) mündet.

4. Wellenanordnung nach Anspruch 1, 2 oder 3, **dadurch gekennzeichnet, dass** die Hülse (19) in Stromrichtung (30) gesehen hinter einem Anschlussraum (28) angeordnet ist, in den eine Entlüftungsöffnung (25) mündet.

5. Wellenanordnung nach Anspruch 1 oder einem der folgenden, **dadurch gekennzeichnet, dass** die Hülse(19) in wenigstens einem Lager (21) gelagert ist, das die Hülse umgibt.

6. Wellenanordnung nach Anspruch 5, **dadurch gekennzeichnet, dass** das Lager (21) ein Wälzlager, ein Gleitlager oder ein hydrodynamisches Lager ist.

7. Wellenanordnung nach Anspruch 1 oder einem der folgenden, **dadurch gekennzeichnet, dass** die Welle (7) eine spindelförmige Außenkontur aufweist.

8. Wellenanordnung nach Anspruch 1 oder einem der folgenden, **dadurch gekennzeichnet, dass** die Welle (7) aus einem Bündel von verdrehten Strängen besteht.

9. Wellenanordnung nach Anspruch 1 oder einem der folgenden, **dadurch gekennzeichnet, dass** die Welle (7) innerhalb einer innerhalb der Hülle angeordneten Wendel (9) verläuft.

10. Wellenanordnung nach Anspruch 1 oder einem der folgenden, **dadurch gekennzeichnet, dass** die Hülle (1) an ihrem dem Antrieb fernen Ende einen Auslass (31,34) für das Fluid aufweist.

11. Wellenanordnung nach Anspruch 10, **dadurch gekennzeichnet, dass** der Auslass eine Durchführung (34) der Welle ist.

12. Wellendurchführung nach Anspruch 10 oder 11, **dadurch gekennzeichnet, dass** das Fluid an dem Auslass (31) aus der Hülle (1) abgesaugt wird.

13. Verfahren zum Betrieb einer Wellenanordnung gemäß einem der Ansprüche 1 bis 12, **dadurch gekennzeichnet, dass** die Welle (7) mit einer Geschwindigkeit von mindestens 300 Umdrehungen pro Minute rotiert.

14. Verfahren zum Betrieb einer Wellenanordnung gemäß einem der Ansprüche 1 bis 12, **dadurch gekennzeichnet, dass** die Welle mit einer Geschwindigkeit von höchstens 40.000 Umdrehungen pro Minute rotiert.

15. Verfahren nach Anspruch 13 oder 14, **dadurch gekennzeichnet, dass** ein konstanter Fluss des Fluids in einen am antriebsseitigen Ende der Hülle (1) gelegenen Anschlussraum (28) gepumpt wird.

16. Verfahren nach Anspruch 13, 14 oder 15, **dadurch gekennzeichnet, dass** das Fluid an einem Auslass (31) (1) an dem antriebsfernen Ende der Hülle aus dieser abgesaugt wird.
